# EUROPEAN PATENT SPECIFICATION

(11) **EP 1 245 218 B1**
(45) Date of publication and mention of the grant of the patent: **26.09.2007**
(21) Application number: 01107597.5
(22) Date of filing: 27.03.2001
(51) Int. Cl.: A61K 6/027, A61K 6/04, A61C 5/08

(54) **A bonding material for joining a porcelain ceramic to a metal framework of a dental restoration or prosthesis**
Bindemittel zum Verbinden einer Porzellankeramik mit einem metallischen Gerüst bei einem Zahnersatz oder einer Prothese
Liant pour joindre une céramique en porcelaine avec une structure métallique pour une restauration dentaire ou une prothèse

(43) Date of publication of application: 02.10.2002
(73) Proprietor: Shoher, Itzhak, Dr., Tel Aviv 61580 (IL); Whiteman, Aharon, Tel Aviv 61580 (IL)
(72) Inventor: Shoher, Itzhak, Dr., Tel Aviv 61580 (IL); Whiteman, Aharon, Tel Aviv 61580 (IL)
(74) Representative: Kinzebach, Werner

(56) References cited:
- EP-A- 0 668 069
- US-A- 4 426 404
- US-A- 4 434 211

## Description

### FIELD OF THE INVENTION

The present invention relates to a bonding method for joining a porcelain ceramic to a metal framework of a dental restoration or a dental prothesis before sintering the porcelain.

### BACKGROUND OF THE INVENTION

It is conventional in the field of restorative dentistry to cover the metal framework of a dental prosthesis or restorative restoration such as a crown or bridge with a polymeric veneering material such as acrylic or a porcelain ceramic to simulate the aesthetics of natural teeth. The veneering material forms the superstructure for the prothesis or dental restoration after it is fused to the underlying metal framework at relatively high temperature. A superstructure of a porcelain ceramic composition may be formed of, for example, natural feldspar, quartz and kaolin and may possibly also include borosilicate glasses, flux and a coloring agent.

EP-A-0 668 069 describes a moldable dental composition which is used to form the metal coping of a ceramic-to-metal or acrylic-to-metal restauration.

To enhance the bond strength between the metal framework and a porcelain superstructure it is conventional practice to apply a bonder to the surface of the metal framework upon which a porcelain ceramic material is coated before firing the porcelain ceramic at relatively high temperature in a dental furnace. A typical bonder contains a composition of finely divided particles of noble metals essentially or entirely of gold. An alternative bonding material which enhances the bond between a porcelain ceramic and a metal framework particularly of a noble metal or alloy composition is described in US Patent No. 4,434,211. In accordance with the aforementioned patent the bonder includes particles composed of a halide of a noble metal in a range of between 1 to 100% by weight of the bonder composition. Although a bonder having a halide of a noble metal results in a clinically unbreakable bond the success in forming a bond of satisfactory bond strength is nevertheless dependent upon firing the porcelain ceramic under controlled conditions in a relatively narrow temperature range.

### SUMMARY OF THE INVENTION

The method of bonding a ceramic material to a dental metal framework is defined in the annexed claim.

A bonding material composed of a precious metal composition with gold as a major constituent has been discovered in accordance with the present invention which will form a clinically unbreakable bond between the metal framework of the dental restoration or prosthesis and a porcelain ceramic superstructure. Moreover, the porcelain can be sintered over a much wider temperature firing range than is presently possible and the bond strength is higher and more consistent over the entire sintering temperature firing range.

Broadly, the bonding material of the present invention comprises a composition of finely divided metal particles having at least 50% gold or gold alloy as the major metal constituent and finely divided particles of activated carbon in a range of from 0.05 wt% to 10 wt% of the weight of the composition. Finely divided metal particles other than gold may be included including non-precious metal particles and/or particles of a noble metal halide.

The bonding material of the present invention should include at least 50 wt% gold or gold alloy and finely divided particles of a carbonaceous material of preferably activated carbon in a range of from 0.05 wt% to 10 wt% of the weight of the composition. Activated carbon is a well-known, porous, carbonaceous material formed by heat-treating carbon or subjecting it to reaction with gases, sometimes adding chemicals, for example, zinc chloride, during or after carbonization, in order to increase its porosity. Its high porosity results in a very high surface area of many orders of magnitude larger than its untreated surface area. The carbonaceous particles from which activated carbon is formed may be of any conventional carbon material, including carbon black, coke flour, calcined lamp black flour, and the like.

Although the activated carbon particles may be present in a range of from 0.05 wt% to 10 wt% of the total bonder composition a more preferred concentration range for activated carbon is between 0.1 wt% and 1.0 wt% of the total bonder composition. The size of the activated particles can vary between 0.5 and 250 microns but preferably at least 70% by weight of the activated carbon particles should have a size of between 5 and 150 microns. The presence of carbonaceous particles, preferably activated carbon, in the bonder composition within the above identified concentration range is essential to the present invention and acts as a solder binding itself to the the metal understructure. This provides for greater latitude in the formation of a clinically unbreakable bond independent of the porcelain ceramic composition and over a wider sintering temperature firing range.

The presence of gold as a major constituent of the binder composition is particularly important to bind a porcelain ceramic to a metal framework of a precious metal composition which contains gold or a gold alloy as a constituent thereof. However it should be understood that the framework may be of any suitable metal or metal alloy composition and is not limited to a metal framework of precious metal(s) or alloys thereof.

The bonder composition of the present invention may comprise, in addition to at least 50 wt% gold or gold alloy and activated carbon, finely divided particles of noble based metal(s) other than gold either in elemental form or as an alloy thereof, a halide of a noble metal and non-precious metal particles with the non-precious metal particles limited in concentration to no more than about 10 wt% of the total bonder composition. If a noble metal halide is included in the bonder composition it should preferably be selected from a halide of a noble metal of silver, platinum, palladium and gold such as, for example, silver chloride or platinum chloride.

The bonder composition of the present invention may be used with or without a suitable binder. If the bonder is suspended in a binder, the binder should be selected to permit the bonder to be easily and readily applied to the surface(s) of the metal framework by any conventional method, such as, brushing, painting, dipping and spraying before the porcelain ceramic material is applied and before the porcelain is sintered. Examples of a liquid binder include water detergents or an organic composition such as alcohol or ethylene glycol. Alternatively, a wax binder may also be used to cover or coat the metal surface(s) over which porcelain is to be applied. The binder selected should be one which will volatilize in the sintering process without leaving a residue. When a binder is not used the finely divided bonder particles may be sprinkled over the metal surface(s) upon which porcelain is to be applied before sintering. There is no coating thickness requirement for the bonder material.

After applying the bonding material of the present invention to the applicable surface(s) of the metal framework over which porcelain is to be applied the metal framework is sintered at a temperature of between 800°C and 1030°C which is a temperature range considerably wider than the sintering temperature range heretofore necessary to produce an acceptable porcelain superstructure of adequate bond strength. The sintering operation causes the porcelain ceramic to fuse to the metal framework through the sintered bonding material which functions as a solder and mitigates for any differences in thermal contraction rates.

## Claims

1. A method of bonding a ceramic material to a dental metal framework of a dental restoration or prosthesis **characterized by**: forming a dental bonding material comprising finely divided metal particles having at least 50% gold or gold alloy as the major constituent thereof and finely divided particles of a carbonaceous material, coating the surface of said dental metal framework with said bonding material, heat treating the coated metal framework at a temperature of between 800 °C and 1030 °C and coating the heat treated surface with a ceramic to produce an acceptable porcelain or other ceramic veneering material superstructure having adequate bond strength.

2. A method as defined in claim 1 wherein said carbonaceous material is activated carbon and is in a concentration range of from 0,05 wt% to 10 wt% of the weight of the composition.

3. A method as defined in claim 2 wherein the heat treatment step is performed in a furnace.

4. A method as defined in claim 3 wherein said bonding material further comprises a binder functioning as a carrier vehicle for the bonder material selected from the group consisting of water detergents and an organic composition and/or a wax.

5. A method as defined in any of the preceding claims wherein said carbonaceous material is activated carbon and is in a concentration range of from 0,1 wt% to 1.0 wt% of the weight of the composition.

6. The method of claim 5 wherein said bonding material further comprises finely divided metal particles of precious metal(s) other than gold in elemental form or as an alloy.

7. The method as defined in claim 6 wherein said bonding material further comprises finely divided metal particles of a noble metal halide.

8. The method as defined in claim 7 wherein said noble metal halide is selected from the group of noble metals consisting of silver, platinum, palladium and gold.

9. The use of a dental material a comprising finely divided metal particles having at least 50% gold or gold alloy as the major constituent thereof and finely divided particles of a carbonaceous material, for forming a clinically unbreakable bond between the metal framework of the dental restoration or prosthesis and a porcelain superstructure.

## Patentansprüche

1. Verfahren zum Verbinden eines keramischen Materials mit einem metallischen Dentalgerüst einer Dentalrestauration oder -prothese, **gekennzeichnet durch**: Bilden eines dentalen Verbindungsmaterials, welches fein verteilte Metallpartikel umfasst, die wenigstens 50 % Gold oder Goldlegierung als Hauptbestandteil und fein verteilte Partikel eines kohleartigen Materials aufweisen, Beschichten der Oberfläche des metallischen Dentalgerüsts mit dem Verbindungsmaterial, Wärmebehandeln des beschichteten metallischen Gerüsts bei einer Temperatur zwischen 800 °C und 1030 °C und Beschichten der wärmebehandelten Oberfläche mit einer Keramik zur Erzeugung einer verträglichen Überstruktur aus Porzellan oder einer anderen Keramik mit ausreichender Verbindungsfestigkeit.

2. Verfahren gemäß Anspruch 1, wobei das kohlenstoffartige Material Aktivkohle ist und in einem Konzentrationsbereich von 0,05 Gew.-% bis 10 Gew.-% bezogen auf das Gewicht der Zusammensetzung vorliegt.

3. Verfahren gemäß Anspruch 2, wobei der Wärmebehandlungsschritt in einem Ofen durchgeführt wird.

4. Verfahren gemäß Anspruch 3, wobei das Verbindungsmaterial außerdem ein Bindemittel umfasst, das als Träger für das Verbindungsmaterial dient und ausgewählt ist aus der Gruppe bestehend aus wässrigen Detergentien und einer organischen Zusammensetzung und/oder einem Wachs.

5. Verfahren gemäß einem der vorhergehenden Ansprüche, wobei das kohlenstoffartige Material Aktivkohle ist und in einer Konzentration von 0,1 Gew.-% bis 1,0 Gew.-% bezogen auf das Gewicht der Zusammensetzung vorliegt.

6. Verfahren gemäß Anspruch 5, wobei das Verbindungsmaterial außerdem fein verteilte Metallpartikel aus von Gold verschiedenem (verschiedenen) Edelmetall(en) in elementarer Form oder als Legierung umfasst.

7. Verfahren gemäß Anspruch 6, wobei das Verbindungsmaterial außerdem fein verteilte Metallpartikel eines Edelmetallhalogenids umfasst.

8. Verfahren gemäß Anspruch 7, wobei das Edelmetallhalogenid ausgewählt ist aus der Gruppe der Edelmetalle bestehend aus Silber, Platin, Palladium und Gold.

9. Verwendung eines Dentalmaterials, das fein verteilte Metallpartikel umfasst, die wenigstens 50 % Gold oder Goldlegierung als Hauptbestandteil und fein verteilte Partikel eines kohlenstoffartigen Materials aufweisen, zur Herstellung einer klinisch unzerbrechlichen Verbindung zwischen einem Metallgerüst einer Dentalrestauration oder -prothese und einer Überstruktur aus Porzellan.

## Revendications

1. Procédé permettant de lier un matériau céramique à une structure métallique dentaire d'une restauration ou d'une prothèse dentaire, **caractérisé par** :
la formation d'un matériau de liaison dentaire comprenant des particules métalliques finement divisées constituées principalement d'au moins 50 % d'or ou d'alliage d'or et des particules finement divisées d'un matériau carboné,
le revêtement de la surface de ladite structure métallique dentaire par ledit matériau de liaison,
le traitement thermique de la structure métallique revêtue à une température comprise entre 800°C et 1030°C et
le revêtement de la surface thermiquement traitée par une céramique afin de produire une superstructure en porcelaine ou autre matériau de plaquette céramique acceptable ayant une intensité de liaison adéquate.

2. Procédé selon la revendication 1, dans lequel ledit matériau carboné est du charbon actif et est compris dans une plage de concentration allant de 0,05 % en poids à 10 % en poids par rapport au poids de la composition.

3. Procédé selon la revendication 2, dans lequel l'étape de traitement thermique est effectuée dans un four.

4. Procédé selon la revendication 3, dans lequel ledit matériau de liaison comprend en outre un liant ayant fonction de véhicule pour le matériau de liaison choisi dans le groupe constitué par les détergents aqueux et une composition organique et/ou une cire.

5. Procédé selon l'une quelconque des revendications précédentes, dans lequel ledit matériau carboné est du charbon actif et est compris dans une plage de concentration allant de 0,1 % en poids à 1,0 % en poids par rapport au poids de la composition.

6. Procédé selon la revendication 5, dans lequel ledit matériau de liaison comprend en outre des particules métalliques finement divisées de métal/métaux précieux autres que l'or sous forme élémentaire ou sous forme d'alliage.

7. Procédé selon la revendication 6, dans lequel ledit matériau de liaison comprend en outre des particules métalliques finement divisées d'un halogénure de métal noble.

8. Procédé selon la revendication 7, dans lequel ledit halogénure de métal noble est choisi dans le groupe des métaux nobles comprenant l'argent, le platine, le palladium et l'or.

9. Utilisation d'un matériau dentaire comprenant des particules métalliques finement divisées constituées principalement d'au moins 50 % d'or ou d'alliage d'or et des particules finement divisées d'un matériau carboné, en vue de former une liaison cliniquement incassable entre la structure métallique de la restauration ou prothèse dentaire et une superstructure en porcelaine.
